Europäisches Patentamt

European Patent Office

Office européen des brevets

(11) Publication number: 0 134 923
A2

## EUROPEAN PATENT APPLICATION

(21) Application number: 84107015.4

(22) Date of filing: 19.06.84

(51) Int. Cl.⁴: **A 61 K 35/14**
A 61 K 37/02, C 12 N 5/00

(30) Priority: 14.07.83 JP 128893/83

(43) Date of publication of application:
27.03.85 Bulletin 85/13

(84) Designated Contracting States:
AT BE CH DE FR GB IT LI LU NL SE

(71) Applicant: YAMANOUCHI PHARMACEUTICAL CO. LTD.
No. 5-1 Nihonbashi-Honcho, 2-chome Chuo-ku
Tokyo(JP)

(72) Inventor: Obara, Tadashi
No. 1-1-15-1007, Kamiochiai
Shinjuku-Ku Tokyo(JP)

(72) Inventor: Ezoe, Hisanori
No. 1-43-8, Uehara
Shibuya-ku Tokyo(JP)

(72) Inventor: Haranaka, Katsuyuki
No. 2-31-10, Hiyoshi-cho
Kokubunji-shi Tokyo(JP)

(72) Inventor: Satomi, Nobuko
No.1-1-11-1103, Ohashi
Meguro-ku Tokyo(JP)

(74) Representative: Wuesthoff, Franz, Dr.-Ing. et al,
Patentanwälte Wuesthoff -v. Pechmann-Behrens-Goetz
Schweigerstrasse 2
D-8000 München 90(DE)

(54) Human endogenous cancer regulatory factor, process for preparing the same, pharmaceutical composition containing the same and human monocytic leukemia cell lines.

(57) A human endogenous cancer regulatory factor (KBS) having a molecular weight of 82,000 ± 10,000 and an isoelectric point of pH 6.5 ± 0.5, a process for preparing KBS and a pharmaceutical composition containing KBS are disclosed. The KBS of the present invention is useful as a potent tumoricidal or anti-tumor agent.

EP 0 134 923 A2

./...

Fig. 1

## TITLE OF THE INVENTION

Human endogenous cancer regulatory factor, process for preparing the same, pharmaceutical composition containing the same and human monocytic leukemia cell lines

## FIELD OF THE INVENTION

The present invention relates to a novel human endogenous cancer regulatory factor, a process for preparing the same, a pharmaceutical composition containing the same, and human monocytic leukemia cell lines.

## BACKGROUND OF THE INVENTION

While several biologically active materials are known to have anti-tumor activities, interferons and tumor necrosis factor (TNF) are most popular. The latter is a proteinic, biologically active material produced by animals, and it is said that unlike interferons, TNF has trans-species potent tumoricidal or anti-tumor activities. Furthermore, the TNF is believed to act on tumor cells both directly and indirectly through the mechanism of immunization in the host.

TNF was first discovered in mice by Old et al. in 1975 at Sloan-Kettering Institute for Cancer Research, U.S.A., and it was later reported by Carswell et al. as a material capable of causing tumor necrosis [Proc. Nat. Acad. Sci. USA, 72, No. 9, pp. 3666-3670 (1975)].

Methods of purifying TNFs (i.e. mouse TNF and rabbit TNF)

- 1 -

0134923

were also reported [Japanese Patent Application Laid-Open Nos. 140725/82 and 140726/82; The Japanese Journal of Clinical Medicine, 40, No. 8, pp. 186-193 (1982)]. These materials are prepared by either of the following two methods: (1) an animal (i.e. mouse or rabbit) is administered with a first stimulant such as formalin-treated Propionibacterium acnes, then with a second stimulant such as lipopolysaccharide from E. coli, and the desired TNF is recovered from the body fluids (e.g. blood) of the animal, and purified; (2) macrophages from an animal that have been activated by a first stimulant are cultured in a tissue culture medium, to which a second stimulant is added to induce TNF which is subsequently recovered and purified.

The biologically active materials obtained by these methods have a molecular weight of 39,000 $\pm$ 5,000 and an isoelectric point of pH 3.9 $\pm$ 0.3. They have excellent anti-tumor activities, and because of their relatively low specificity to the species and their virtual nontoxicity, these materials are considered to be the most promising anti-tumor agents.

In recent years, methods for producing TNF from human cells have been reported (Japanese Patent Application Laid-Open Nos. 21621/83 and 107197/83). There is no knowing how this human TNF differs from TNF from animals such as mice and rabbits because these patent applications give no physicochemical data on the obtained human TNF such as its molecular weight and isoelectric point. However the, corresponding British Patent Application Laid-Open No. 2,106,117 states that the physicochemical data of the human TNF produced was in good agreement with that of the mouse TNF reported by Carswell et al., and therefore, it is highly likely that this human TNF is the same as the animal TNF.

## SUMMARY OF THE INVENTION

As a result of various studies made under such prior art, the inventors have found that a novel biologically active material can be produced by growing normal human or leukemic cells in a tissue culture medium, then adding 12-0-tetradecanoyl phorbol-13-acetate (TPA) and lipopolysaccharide from $\underline{E.\ coli}$ to the medium in the order written. The new biologically active material is more effective, either in vitro or in vivo, than mouse or rabbit TNF against many kinds of tumors including human tumor cells. Furthermore, the physicochemical properties, such as the molecular weight, isoelectric point and stability, of this new biologically active material significantly differ from those of the known animal and human TNF.

The biologically active material of the present invention is a potent tumoricidal material derived from human cells, and in addition, because of its ability to cause tumor necrosis, this material is considered to be one of the substances that are produced in very small amounts in the body of a patient who has spontaneously recovered from a cancer. Therefore, we named this material a human endogenous cancer regulatory factor (hereunder referred to as KBS).


## BRIEF DESCRIPTION OF THE DRAWINGS

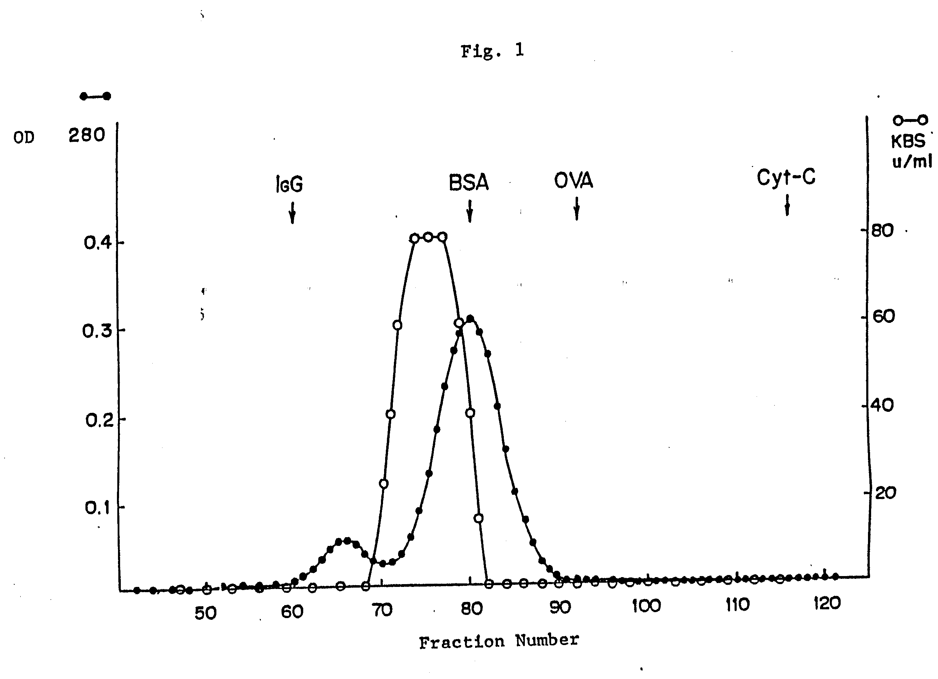

Fig. 1 shows an elution profile of column chromatography on Sephadex G-200 performed in Example 1, in which the arrow ($\longrightarrow$) indicates the each reference protein, $I_G G$ refers to rabbit IgG (mol. wt. 160,000), BSA refers to bovine serum albumin (mol. wt. 67,000), OVA refers to ovalbumin (mol. wt. 43,000), Cyt-C refers to cytochrome C (mol. wt. 13,000), the closed circles ($\bullet\!\!-\!\!\bullet$) indicate the UV-absorption at 280 nm, the open circles ($\circ\!\!-\!\!\circ$) indicate the cytotoxic activity unit (U/ml) of KBS against L cells, and the horizontal axis plots the fraction number.

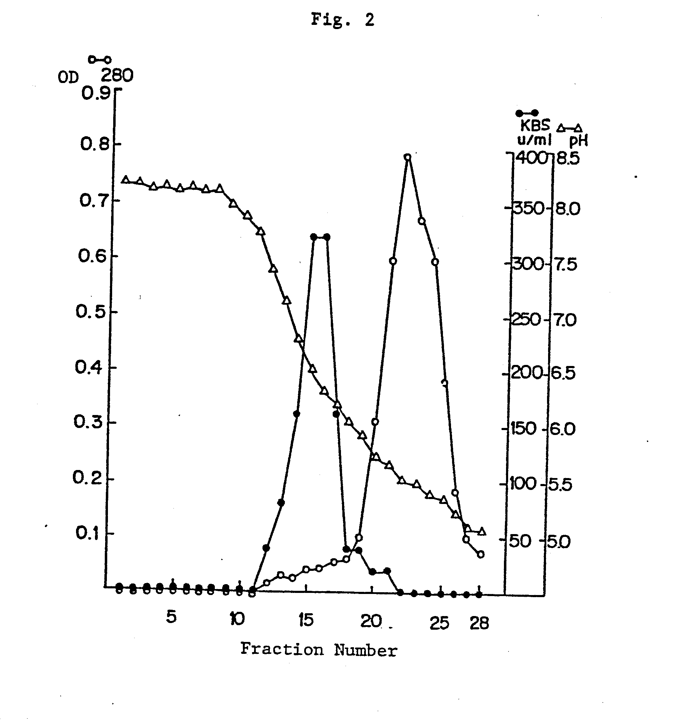

Fig. 2 shows an elution profile of chromatofocusing by high-performance liquid chromatography conducted in Example 1, in which the open circles ($\circ\!\!-\!\!\circ$) indicate the UV-absorption at 280 nm, the closed circles ($\bullet\!\!-\!\!\bullet$) indicate the cytotoxic activity (U/ml) of KBS against L cells, the open triangles ($\triangle\!\!-\!\!\triangle$) indicate pH, and the horizontal axis plots the fraction number.

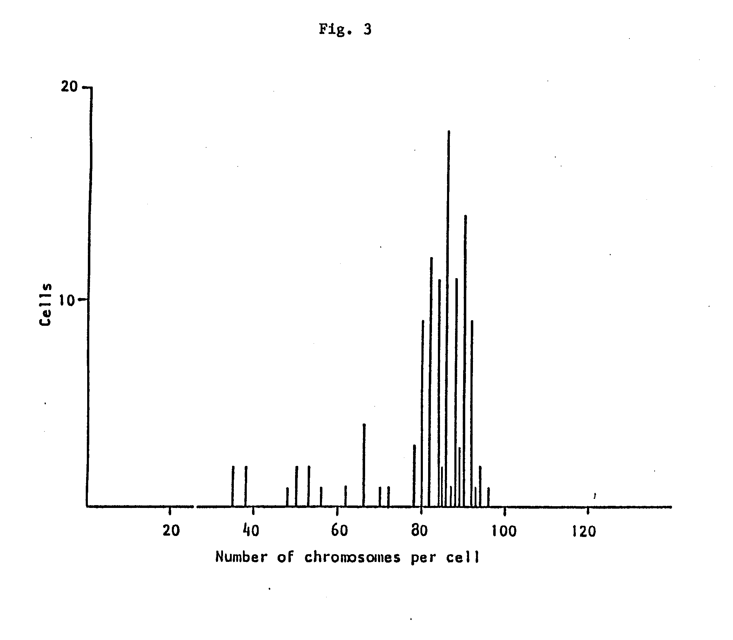

Fig. 3 shows a distribution of chromosomes of YKBS-7-15, in which the longitudinal axis plots the number of cells and the horizontal axis plots the number of choromosomes ph cell.

DETAILED DESCRIPTION OF THE INVENTION

The KBS of the present invention is produced by growing normal human or leukemic cells in a tissue culture medium in the presence of a first stimulant, adding a second stumulant to the medium so as to produce KBS, and recovering said KBS.

The normal human cells or leukemic cells as used in the present invention include normal monocyte and macrophage cells, as well as their malignant cells. Any of these cells can be preferably used so long as they can be readily proliferated in a tissue culture medium. Particularly preferred are monocyte/macrophage series of leukemia cells such as HL-60; monocytic leukemia cells selected from clinically established peripheral leukocytes of monocytic leukemia origin such as YKBS-7-15, YKBS-7-16 and YKBS-7-17; and monocloned or subcloned cell lines obtainable from these cell lines by a conventional manner. The cultured cell lines YKBS-7-15, YKBS-7-16 and YKBS-7-17 are the monocytic leukemia cell lines isolated and established by the present inventors by culturing the buffy coat leukocytes from the peripheral blood of a patient with acute monocytic leukemia according to the conventional method (i.e. removal of contaminating erythrocytes by treating with ammonium chloride).

Other examples of the monocyte/macrophage series of cell lines include Mono-1, Mono-1-207 [Virchow Arch. A. Pathol. Anat. Histol., _371_, 15 (1976) and _379_, 269 (1978)]. Cells capable of differentiating into the cells with properties as described above may of course be used in the present invention. These cells eventually exhibit properties of monocytic leukemia, and malignant myeloid cells are one example.

The cells listed above are usually subjected to tissue culture in synthetic nutrient media containing fetal calf serum. For instance, YKBS-7-15 cell line used in Example 1 to be described later in this specification is cultured in RPMI medium (Gibco Co.) containing FCS (fetal calf serum), HEPES and one or more antibiotics. Synthetic serum-free nutrient media can also be used for tissue culture of these cells. The cells may also be proliferated intraperitoneally or subcutaneously·in no-human warm-blooded animals such as nude mice and juvenile hamsters. The tissue culture medium as used in the present application includes such _in vivo_ growth media.

Examples of the first stimulant used in the present invention include conditioned media with lymphocytes or lymphoblasts, chemical substances or natural extracts that induce leukocytes by differentiation, as well as mixtures thereof. Another example is the supernatant produced by culturing immunization-mediating cells in animals administered with a certain chemical substance such as Trichothecene mycotoxin. One example of the chemical substance or natural extract that induces leukocytes by differentiation is phytohemagglutinin (PHA), but macrophage activating substances such as muramyl dipeptide (MDP), 12-0-tetradecanoyl phorbol-13-acetate (TPA), 12,13-phorbol butyrate, dimethylsulfoxide (DMSO) and mezerein are particularly preferred.

Substances capable of activating the reticuloendothelial system may also be used as the first stimulant in the present invention. Common examples include Gram-positive bacteria, fungus-produced materials, protozoans and yeasts, and these are used as live cells, dead cells (after heat- or formalin-treatment ) or cell extracts. Illustrative Gram-positive bacteria include Propioni bacteria such as Propionibacterium acnes (Corynebacterium parvum) and Propionibacterium granulocum (Corynebacterium granulocum); Mycobacteria such as Bacillus Calmette-Guerin (B.C.G.) and Mycobacterium smegmatis; and Nocardia such as Nocardia erythropolis and Nocardia gardnerl. Illustrative fungus-produced materials are toxins produced by Fusarium. Typical protozoan include Plasmodium and Toxoplasma gondii. A commonly employed yeast is Zymosan extracted from Saccharomyces cerevsiae or the like. Synthetic polymers such as pyran copolymers may also be used as the first stimulant.

The second stimulant used in the present invention is an endotoxin produced from Gram-negative or Gram-positive bacteria. Illustrative endotoxins include lipopolysaccharides derived from E. coli, Pseudomonas aeruginosa and typhoid bacillus. Lipopolysaccharides from Gram-positive bacteria can also be used with good results.

The KBS of the present invention can be produced by growing normal human cells or human monocytic leukemia cells in a conventional tissue culture medium comprising a synthetic nutrient medium, serum, etc., with the first stimulant being added before, during or after the growth of these monocytic leukemia cells, and then adding the second stimulant to the culture medium so as to induce the desired KBS. For the induction of KBS from the human monocytic leukemia cells the two stimulants are usually utilized but even one of stimulants will serve the purpose.

In any event, the practical method comprises the following steps: 1) normal human cells or human monocytic leukemia cells are fully grown in a common tissue culture medium; 2) the first stimulant, e.g. 12-0-tetra- decanoylphorbol-13-acetate (TPA), is added to the medium in an amount of, say, 0.1 - 100 ng/ml to effect the first stimulation; 3) following a continued cultivation for a period of, say, 12 hours to 3 days, the second stimulant, e.g. lipopolysaccharide from E. coli, is added to the medium in an amount of, say 0.1 - 10 µg/ml to effect the second stimulation; and 4) the cultivation is further continued for a period of, say, 8 to 24 hours until KBS is induced in the supernatant of the culture.

The KBS thus produced can be recovered after separation and purification by known techniques such as dialysis, salting-out, filtration, centrifugation, concentration and lyophilization. If further purification is necessary, these techniques may be combined with adsorption onto an ion exchanger and elution therefrom, gel filtration, affinity chromatography, isoelectric fractionation, electrophoresis, or high-performance liquid chromatography (HPLC). Usually, the supernatant containing the desired KBS is separated from the culture by centrifugation or any other suitable method, and the separated supernatant is purified by adsorption onto a basic anion exchanger. Suitable basic anion exchangers include DEAE-Sephadex A-50, DEAE-Sepharose CL-6B, DEAE-Sephacel and QAE-Sephadex A-50 (these are products of Pharmacia AB), AIEC DE 52 (Wattman Co.), Servacel AE (Serva Co.) and Cellex QAE (Bio-rad Laboratories).

When the KBS-containing supernatant is subjected to a column filled with, e.g. DEAE-Sephadex A-50, the KBS is transferred into non-adsorbed fractions and most of the other proteins are adsorbed on the gel. After concentrating the non-adsorbed fractions with, e.g. polyethylene glycol 6,000 or by ultrafiltration, e.g. using Amicon Hollow Fiber system (Amicon Co.), followed by optional dialysis, the concentrated fractions are again subjected to a DEAE-Sephadex A-50 column, whereupon the KBS is adsorbed on the gel. The adsorbed KBS is then eluted with 0.02M tris-HCl buffer solution (pH 7.8) containing 0.19M sodium chloride so as to recover KBS in a purified form. Alternatively, crude KBS may be directly adsorbed onto DEAE-Sephadex A-50 having a higher gel content, and the adsorbed KBS is eluted with 0.02M tris-HCl buffer solution (pH 7.8) containing 0.19M sodium chloride. If further purification of KBS is necessary, high-performance liquied chromatography, gel filtration, affinity chromatography, chromatofocusing and slab electrophoresis with polyacrylamide gel may be used. Among these techniques, ion exchanging or chromatofocusing recently developed using HPLC chromatographic devices (FPLC Mono-Q and Mono-P by Pharmacia AB) is particularly suitable for achieving a higher degree of purification.

The KBS of the present invention produced, recovered and purified by the methods described above has the following physicochemical properties.

(1)  Molecular weight

The KBS has a relative molecular weight of 82,000 $\pm$ 10,000 as measured by molecular sieve using Sephadex G-200 (Pharmacia AB).

(2)  Isoelectric point

The KBS has an isoelectric point of pH 6.5 $\pm$ 0.5 as measured by chromatofocusing using a HPLC chromatographic device (FPLC Mono-P by Pharmacia AB).

(3)  Stability against pH changes

According to equilibrium dialysis (room temperature x 17 hr), an aqueous solution of the KBS was stable in the pH range of 5.0 - 10.0, but was somewhat unstable at a pH below 4.5 or above 10.5.

(4)  Heat stability

The KBS remained stable in an aqueous solution even after it was heated at 56°C for 30 minutes.

The KBS of the present invention is a novel biologically active material which differs significantly from both mouse and rabbit TNF in regard to the molecular weight, isoelectric point and stability.  Anti-rabbit TNF-containing mouse anti-serum reacted almost completely with rabbit TNF, and partially with mouse TNF, so as to inhibit the activities of TNF.  However, the anti-serum hardly reacted with the KBS and the activities of TNF could not be inhibited.

This fact, together with the above mentioned differences in physicochemical data, indicates that the KBS of the present invention differs also immunologically from TNF.

Unlike TNF which is derived from animals such as mice and rabbits, the KBS of the present invention is a biologically active material derived from humans. Therefore, it is not considered a foreign material by the human body and has the least possibility of causing an antigen-antibody reaction.

The effectiveness of the KBS of the present invention against human tumor cells such as Hep-2, Kato-III, KB, Hela, HL 60 and SEKI is much greater than that of TNF, and in an in vivo test using mouse-derived tumors (Meth A), the KBS proved to be as potent as or more potent than TNF in its tumor necrosis and anti-tumor activities.

The KBS of the present invention has the following anti-tumor actions.

I.   Cytotoxicity (in vitro)

A suspension of $5 \times 10^6$/ml cells in a culture medium (90% MEM + 10% FCS) was transferred into wells in a microplate in an amount of 250 µl/well.  An equal volume of KBS diluted with a culture medium (90% MEM + 10% FCS) was added to the suspension in each well, and the mixture was incubated at 37°C for 48 hours.  Thereafter, the incubated cells were recovered from the microplate and the number of live cells was counted by the dye exclusion test using a 0.5% solution of trypan blue.  The cytotoxic effect of the KBS was evaluated by the percentage  cytotoxicity  of the treated group against the control group.  The cytotoxicity data for KBS and that of mouse TNF are given in Tables I and II, respectively.  The cell lines used in the cytotoxicity test were Hep-2, Kato-III, KB, Hela, HL 60 and SEKI as human cancer cells, and Chang Liver as normal human cells.

Table I  Cytotoxic Effects of KBS

| cytotoxicity (%) / Cell line | KBS (U/ml) | | | | | | |
|---|---|---|---|---|---|---|---|
| | 8,000 | 4,000 | 2,000 | 1,000 | 500 | 250 | 125 |
| Hep - 2 | 84.4 | 61.9 | 44.2 | 32.9 | 8.9 | 15.4 | – |
| Kato - III | 62.0 | 68.9 | 60.2 | 54.9 | 33.9 | 37.2 | 21.9 |
| K B | – | – | 59.9 | 38.3 | 13.8 | 0 | 0 |
| Hela | – | – | 76.8 | 28.1 | 12.3 | 16.0 | 5.8 |
| HL 60 | – | – | 74.9 | 50.0 | 26.3 | 15.7 | 18.9 |
| SEKI | – | – | 67.1 | 43.7 | 20.9 | 13.8 | 0 |
| Chang Liver | – | – | 30.8 | 10.9 | 0 | 0 | 0 |

Table II  Cytotoxic Effects of Mouse TNF

| cytotoxicity (%)  Cell line | Mouse TNF (U/ml) | | | | | | | | | |
|---|---|---|---|---|---|---|---|---|---|---|
| | 128,000 | 64,000 | 32,000 | 16,000 | 8,000 | 4,000 | 2,000 | 1,000 | 500 | 250 |
| Hep - 2 | 81.7 | 82.0 | 81.7 | 59.8 | 39.6 | 25.5 | 14.4 | 9.6 | 8.0 | 6.6 |
| Kato - III | 87.0 | 85.8 | 85.8 | 54.1 | 38.4 | 24.4 | 22.8 | 19.1 | 15.2 | 5.0 |

0134923

As the above tables show, the KBS of the present invention has cytotoxic effects on human cancer cells and is much more potent than mouse TNF. On the other hand, the KBS of the present invention has substantially no cytotoxic effects on normal human cells.

II. Tumor necrosis activity (in vivo)

Cells ($5 \times 10^5$) of mouse-derived Methylchoranthrene A induced sarcoma (Meth A) serially maintained with Balb/C mice were transplanted into the dorsal skin of Balb/C mice (7-week-old and male). Seven days later, the tumors developed were measured for their size. Then, the mice were injected intravenously with 0.5 ml of KBS diluted stepwise in the range of between 3,200 and 400 units. Twenty-four hours later (8 days after the Meth A transplantation), the tumors were checked for the occurrence of any necrosis, and 48 hours later (9 days after the Meth A transplantation), the tumor size was measured. The degree of tumor necrosis was determined by the method of Haranaka et al. [The Japanese Journal of Clinical Medicine, 40, No. 8, pp. 186-193 (1982)], and the tumor size measurement was directed only to the diameter.

The results were as follows. The group administered 800 to 3,200 units of KBS saw the onset of tumor necrosis (+) 24 hours after the administration. Forty-eight hours later, the necrosis progressed slowly as indicated by a change

16

0134923

from (+) to (++) and from (-) to (+) or (++). In the group administered 800 to 3,200 units of KBS, the tumor size apparently decreased 48 hours later, and all mice in the group administered 3,200 units of KBS became completely normal. The tumor size continued to decrease in all cases that first saw a tumor size reduction on the 48th hour of the KBS administration, and when 10 days passed (17 days after the Meth A transplantation), the transplanted Meth A came off and the mice had become completely normal. In the control group, the tumor diameter became 1.5 cm or more on the 17th day of the Meth A transplantation, and on the 40th day and thereafter, almost all mice were dead.

The above results show that the KBS of the present invention has great tumor necrosis action and potent anti-tumor effects. The KBS of the present invention exhibits particularly potent anti-tumor activity against Sarcoma 180, Ehrlich solid tumor, P 388 solid tumor and Lewis Lung Carcinoma.

The biological activity (KBS activity) used as an index for the screening of possible KBS materials was evaluated by in vitro cytotoxicity testing with normal or mammalian cancer cells. The testing was conducted in accordance with the method of Carswell et al. [Proc. Nat. Acad. Sci., USA, 72, No. 9, pp. 3666-3670 (1975)] using a culture plate of Lympro Co., USA, Eagle's minimum essential

medium (MEM medium) containing non-essential amino acids, 10% of thermally inactivated fetal calf serum, 100 units/ml of penicillin and 100 µg/ml of streptomycin, and L cells (S). A suspension of $1 \times 10^5$ cells and an equal volume of a diluted sample are incubated on the MEM medium at 37°C for 48 hours in an atmosphere containing 5% $CO_2$. The titer is calculated by plotting the degree of sample dilution against 50% cytotoxicity of L cells. The biological activity necessary to kill 50% of the L cells is referred to as one unit (U).

When the KBS prepared by the present invention is used as a medicine, it is administered in a form suitable for exhibiting the intended anti-tumor effects. The KBS may be immediately administered as a medicine, but according to the pharmaceutical practice, it is more commonly presented as a mixture with pharmaceutically acceptable diluents and/ or other pharmacologically effective substances. Therefore, the KBS of the present invention is capable of assuming the proper form for oral or parenteral administration. Illustrative formulations are powders, granules, tablets, sugar-coated tablets, capsules, pills, suppositories, suspensions, liquids, emulsions, injections and aerosols. Typically, the KBS is lyophilized until use when it is dissolved in physiological saline, sterile water or aseptic isotonic solution for injections, and the resulting solution is injected into humans intravenously, subcutaneously or intramuscularly. Before the lyophilization, the KBS may be mixed with suitable stabilizers such as mannitol and human serum albumin, as well as solubilizing agent such as glycine.

The dose of the KBS of the present invention depends on various factors such as the patient's sensitivity, age, sex, body weight, mode of administration, its timing, interval, severity of the disease, the patient's condition, the nature and kind of each formulation. Therefore, the dose level specified below should be taken merely as a guide figure, and usually, smaller doses will adequately serve the purpose, and in some cases, greater doses may become necessary. The minimum dose level for adults is 10,000 units/day.

More importantly, the present invention includes a basic technology for producing KBS by genetic engineering techniques. First, human cells or human monocytic leukemia cells are grown in a tissue culture medium by the method described above, and after the first and second stimulations, the grown cells are incubated for an additional 8 to 24 hours. The cells separated by centrifugation are washed thoroughly with an isotonic buffer solution, either destroyed by the guanidium thiocyanate /mercaptoethanol method or dissolved in a detergent (e.g. Triton N101), and treated with phenol or the like to extract RNA. The RNA extract is passed through an Oligo dt-cellulose column to extract KBS mRNA, which is fractionated by sucrose density gradient centrifugation. The resulting KBS mRNA fractions are introduced into hormonized Xenopus laevis oocytes with a micro injector. After incubating the oocytes on a medium for a predetermined period, the medium or the oocytes are homogenized and subjected to centrifugation. The supernatant obtained is subjected to the determination of KBS activity in terms of cytotoxic activity against L cells, and fractions having higher KBS activity are collected as crude KBS mRNA.

Fractions containing KBS mRNA of higher specificity can be obtained by first treating monocyte / macrophage series of leukemia cells, such as YKBS -7-15, in the same manner as above, extracting KBS-producing polysome from the cells in a specific manner by the antibody affinity method, and passing the extracted polysome through an Oligo dt-cellulose column.

The KBS mRNA prepared by either method may be converted to cDNA by a suitable reverse transcriptase, and a cloning vector such as pBR 322 harboring the cDNA is introduced into a host bacterium so as to transform it, and by the conventional positive or negative screening technique, the desired KBS-specific mRNA can be obtained or purified as the associated cDNA. By this procedure, the intended KBS can be produced from host cells which contain a recombinant cloning vector carrying DNA segments coding for the KBS.

The present invention is hereunder described by working examples which are given here for illustrative purposes only and are by no means intended to limit the scope of the invention.

Formulation (injection)

A million units of the KBS of the present invention was dissolved in 100 ml of physiological saline, and the solution was aseptically passed through a membrane filter. One-milli liter aliquots of the filtrate were put into sterile vials and lyophilized.

Example 1

YKBS-7-15 (monocytic leukemia cells) which was cultured cells derived from peripheral buffy coat leukocytes of human monocytic leukemia were incubated in 700 ml of RPMI 1640 medium with 10% fetal calf serum (a synthetic nutrient medium commercially available from Gibco Co.) at $37^{O}C$ in an atmosphere containing 5% $CO_2$ for a sufficient period to produce $5.0 \times 10^5$ cells/ml. Thereafter, 2 ng/ml of 12-0-tetradecanoyl phorbol-13-acetate (TPA) was added as the first stimulant, and one day after the start of the incubation, 1 g/ml of an endotoxin (lipopolysaccharide derived from E. coli 0111; B4) was added as the second stimulant. After 16 hours of the

- 20 -

continued incubation, the culture medium was centrifuged to recover a supernatant containing the desired KBS.

The supernatant was subjected to a DEAE-Sephadex A-50 column ($3^\phi \times 25$ cm) that had been equilibrated with 0.02M tris-HCl buffer (pH 7.8) containing 0.04M NaCl. The ion exchanger was thoroughly washed with the same buffer at a flow rate of 20 ml/hr to recover the unadsorbed fractions. These fractions were concentrated with polyethylene glycol 6,000 and thoroughly dialyzed against the same buffer. The dialyzed supernatant was again passed through a DEAE-Sephadex A-50 column ($3.2^\phi \times 10.5$) cm) that had been equilibrated with the same buffer. The ion exchanger was thoroughly washed with the same buffer and the adsorbed active fractions (KBS) were eluted with 0.02 M tris-HCl buffer (pH 7.8) containing 0.19M NaCl. The recovered KBS had a titer of $4.6 \times 10^4$ units in terms of cytotoxicity against L cells. Parts of the active fractions was passed through a molecular sieve made of Sephadex G-200 (Pharmacia AB) column ($2.8^\phi \times 90$ cm). The result showed that the KBS produced had a relative molecular weight of $82,000 \pm 10,000$. The elution profile of the chromatography on Sephadex G-200 column is shown in Fig. 1.

The reference proteins used were rabbit IgG ($I_GG$: mol. wt. 160,000), bovine serum albumin (BSA: mol. wt. 67,000), ovalbumin (OVA: mol. wt. 43,000) and cytochrome C

0134923

(Cyt-C: mol. wt. 13,000). The column size was 1.5 x 87 cm and 1 ml of fractions were collected after elution with 0.02M tris-HCl buffer (pH 7.8) containing 0.7M NaCl at a flow rate of 14 ml/hr.

Part of the above active fractions was subjected to high-performance liquid chromatography with an ion exchange column (FPLC Mono-P column by Pharmacia AB) and upon elution by chromatofocusing, the active fractions were recovered as a single sharp peak at pH 6.5 ± 0.3. The result chowed that the KBS prepared by the Example had an isoelectric point of 6.5 ± 0.3. The elution profile of the chromatofocusing by HPLC is shown in Fig. 2. The column used was Mono-P (0.5 x 20 cm) and 2 ml of fractions were collected after elution by linear density gradient (pH 8.3 - 5.0) with Poly buffer (pH 5.0, product of Pharmacia AB) at a flow rate of 0.5 ml/min. The average specific activity of the KBS was 8,000 units per mg protein.

The stability of the active fractions against pH changes was determined by subjecting samples of a given amount of equilibrium dialysis, wherein the samples were dialyzed at room temperature for 17 hrs against buffers whose pH varied from 2.5 to 10.5 by an increment of unity and the residual activity was measured. In the pH range of 5.0 to 10.0, the KBS was completely stable with 100% residual activity, but below 4.5 or above 10.5, the KBS was slightly unstable. The results are also shown in Table 1.

### Table 1

| pH | Residual KBS activity (U/ml) | Relative activity (%) |
|---|---|---|
| Control (untreated) | 2,560 | 100 |
| 2.5 | 20 | 0.8 |
| 3.5 | 640 | 25 |
| 4.5 | 1,260 | 50 |
| 5.5 | 2,560 | 100 |
| 6.5 | 2,560 | 100 |
| 7.5 | 2,560 | 100 |
| 8.5 | 2,560 | 100 |
| 9.5 | 2,560 | 100 |
| 10.5 | 1,280 | 50 |

The active fractions had 100% residual KBS activity even after they were heated at 56°C for 30 minutes.

### Example 2

Cultured cells YKBS-7-15 ( monocytic leukemia cells) were subjected to spinner culture in a serum-free medium HB101 TM (2,000 ml, a medium for tissue culture produced by Hana Biologics Co.) at 37°C for a sufficient period to produce $5.0 \times 10^5$ cells/ml. Thereafter, 2 ng/ml of 12-0-tetradecanoylphorbol-13-acetate (TPA) was added as the first stimulant, and 36 hours after the start of the incubation, 1 µg/ml of an endotoxin (lipo-polysaccharide derived from E. coli 0111; B4) was added as the second stimulant. Following incubation for an additional 16 hours, the KBS containing supernatant was separated from the culture by centrifugation.

As in Example 1, the supernatant was passed through a DEAE-Sephadex A-50 column to recover the active fractions. The titer of the KBS of the present invention was $1.2 \times 10^5$ units in terms of cytotoxicity against L cells. Part of the active fractions was subjected to HPLC with an ion exchange column (FPLC Mono-P column by Pharmacia AB) and the KBS active fractions were recovered by chromatofocusing. The fractions had a specific activity of 80,000 units/mg protein.

0134923

The human monocytic leukemia cell line YKBS-7-15 employed in the above Examples is a novel cell line which has not yet been known, and since this has now been found possible to be permanently continuously cultured, it was named "YKBS-7-15" and deposited with the Collection Nationale de Cultures de Microorganismes (C.N.C.M.), Pasteur Institute, France as No. I-258

Heretofore, of the human monocytic leukemia cell line, cell lines which can be continuously cultured have been hardly known, and the cell line "YKBS-7-15" which the present inventors have now successfully isolated and established is one of the very few and valuable successful examples. The present cell line is quite stable in its proliferating and various other properties, and it could be unlimitedly cultured, and not only produces KBS which is expected as a potent tumoricidal or anti-tumor agent as described above but also may be employed as a parent cell line in the course of production of hybridoma by cell fusion with other cell lines.

The method by which the present cell line, YKBS-7-15 has been isolated and established, and its various properties are described below.

Isolation and Establishment of Cell Line

Ten ml of heparinized peripheral blood, obtained in September, 1982, from an acute leukemia patient (male, 53 years old) diagnosed as M4 according to FAB Classification was centrifuged to isolate peripheral buffy coat leukocytes.

Ten ml of 17 mM Tris buffer (pH 7.6) containing 140 mM ammonium chloride was added to these peripheral buffy coat leukocytes, centrifuged and the process was repeated three times to remove erythrocytes. Thus obtained cell pellet was washed with a serum-free RPMI 1640 medium (Gibco Co.) and centrifugation, and then resuspended in an RPMI 1640 medium containing 10% FCS (Flow Laboratories), 1 mM HEPES (Gibco Co.), 50 I.U./ml penicillin, 50 µg/ml streptomycin and 1 µg/ml tylosin, and incubated in a 25 $cm^2$ plastic culture flask in the atmosphere of 5% carbon dioxide and 95% air at 37°C. One day later, the half of the volume of the culture medium was replaced by a fresh RPMI 1640 medium containing 10% FCS (Flow Laboratories), 15 mM HEPES (Givco Co.), 5 mg/l kanamycin and 5 mg/l streptomycin (2-fold dilution cultrure). Thereafter, the medium change was conducted every 2 or 3 days in the same manner as above to establish a monocytic leukemia cell line "YKBS-7-15" which can grow continously in culture.

Medium for Maintaining and Proliferating Cells

This cell line can be maintained and proliferates satisfactorily in an RPMI 1640 medium (Gibco Co.) containing 10% FCS (Flow Laboratories), 15 mM HEPES (Gibco Co. or Wako Junyaku), 50 mg/l kanamycin and 50 mg/l streptomycin. The antibiotics used above are not limited to kanamycin and streptomycin but other antibiotics such as penicillin, gentamicin, sisomicin etc. may be also employed. The present cell line can be also maintained and proliferates satisfactorily in a serum-free medium specified for tissue culture incorporating the above antibiotics, for example, HB 101$^{TM}$ medium (Hana Biologics Co.) or ISCOVE's medium (Flow Laboratories).

## Maintaining and Proliferating Conditions for Cells

This cell line proliferates satisfactorily under temperature between 36 - 38°C, preferably 37°C, and under pH conditions of 6.5 - 7.5, preferably 7.2. Incubation in an incubator containing 5% carbon dioxide and 95% air is suitable.

## Proliferating Ability of Cells

When $1 \times 10^5$ cells/ml of this cell line is incubated under the above-described conditions, it reaches a cell density of at least $3 - 5 \times 10^5$ cells/ml in 2 to 3 days.

## Continuous Culture

This cell line can be cultured continuously and indefinitely. At present, this has been cultured for more than a year and prceeds the 123rd continuous culture stage.

## Preservation in Frozen Form

This cell line can be frozen to -80°C or beneath under certain conditions such as using a Programme Freezer (Cryo-Med Co.) and stably stored in a freezer or in liquid nitrogen freezing container for a long time without remarkable decrease in the surviving cells.

## Cytological Propeties

## Morphological Properties

Under light microscopic observation, this cell line has a typical shape of monocytic cells in appearance showing spherical or irregularly spherical shape, with the size fluctuating depending on the incubation conditions. Cytoplasm, nuclei, nucleoli etc. are clearly distinguished.

In addition, when a ultra-thin specimen of the present cells is examined by an electron microscope, an ordinary pleomorphic appearance with various sizes of cytoplasmic organs such as mitochondria, well-developed rough endoplasmic reticulum and Golgi's apparatus are observed. Microvilli-like protrusions are also observed at the surface of cell membrane.

Chromosome analysis

(1) Light microscopic observation of chromosomes revealed that chromosome of this cell line did not have a normal euploidy but an abnormal aneupoidy.

(2) Chromosomes number

The cell was incubated in the presence of colchicine of a concentration of 50ng/ml at 37°C under 5% $CO_2$ for 2 hours, then treated with a hypotonic solution (0.075 M KCl : 1% sodium citrate = 4 : 1) at 37°C for 20 minutes, and fixed on a slide glass by using a fixing solution (acetic acid : ethanol = 1 : 1) at room temperature, and further subjected to Giemsa's staining. Photograph was taken for each preparation. The number of chromosomes was conducted on 115 samples prepared from the cells in the metaphase. As the result, the numbers of the nuclear chromosomes of the respective cells was shown to distribute in the range between 35 and 96 as shown in Fig. 3 and the peak was present at about 86.

## Cytochemical Properties

By histochemical staining, this cell line was shown to be negative with Sudan black, peroxidase and alkaline phosphatase [J. Clin. Path. Bact., 59, 336 (1947), Jap. J. Clin. Path., 48, 27 (1982) and 18, 41 (1970)]. Most of the cells are positive with periodic acid - Schiff, PAS and β-glucuronidase [ Jap. J. Clin. Path., 48, 43 and 126 (1982)], although some being strong and other being weak. The cells are also strongly positive with naphthol AS - D chloroacetate esterase, α-naphtyl butyrate esterase and acid phosphatase [J. Histochem. Cytochem., 21, 1 (1973) and Rinsho Kensa, 20, 181 (1976)].

## Cell Marker

(1)  Cell Surface Marker

Each two hundred μl portions of monoclonal antibodies (OKM1, OKIa 1 and OKT3 were used after 40-fold dilution of the stock solution and the stock solutions of anti-Mo2, anti-I2 and anti-J5 were used as they were) was added to 1 x $10^6$ cells of the cell line. They were incubated in an ice bath for 30 minutes, washed and reacted with FITI-labeled goat anti-mouse immunoglobulin (Cappel Co.). The cell surface antigens of this cell line were examined by the indirect fluorescence antibody technique. As the result, this cell line was shown to be negative with anti-monocyte antibody [OKM1: J. Immunol., 124, 1943 (1980); anti-Mo2: J.

Immunol., 126, 1435 (1981)], anti-peripheral T lymphocyte antibody [OKT3: Transplant. Proc. XII (suppl. 1), 141 (1980)] and common acute lymphoblastic leukemia antigen-recognizing antibody [anti-J5: Nature, 283, 583 (1980)] and positive with anti-DR framework antibody [OKIa 1: J. Exp. Med., 150, 1472 (1979); anti-I2: Human Immunol., 1, 77 (1981)]. The employed monoclonal antibodies were obtained from Ortho Diagnostic System Co. (OKM1, OKIa 1 and OKT3) and from Coulter Co. (anti-Mo2, anti-I2 and anti-J5).

Further, the surface immunoglobulin of this cell line was examined by direct immunofluorescence antibody technique using FITI-labeled goat anti-human immunoglobulin H-chain (anti-$\gamma$, anti-$\mu$, and anti-$\alpha$) and L-chain (anti-$\kappa$ and anti-$\lambda$) (Cappel Co.), but it was all negative.

(2) Rosette Formation

Rosette formation was examined for sheep erythrocytes (E), antibody-sensitized sheep erythrocytes (EA) and antibody complement-sensitized erythrocytes (EAC). The results indicated that 1% or less, about 5% and about 15% of the cells were positive in Rosette formations with each erythrocyte respectively.

(3) Cytoplasmic Immunoglobulin

Cytoplasmic immunoglobulin was examined by direct immunofluorescence antibody technique using FITI-conjugated goat anti-human immunoglobulin. The result indicated that about 30% of the cells were positive to IgG ($\gamma$-chain and $\kappa$-chain).

## Phagocytosis

After addtion of FITI-labeled latex particles or Indian ink, the cells were incubated at 37°C in 5% $CO_2$ for 2 hours, washed, and the smear preparations of 500 or more cells were prepared for each specimens respectively. The former was directly examined under a fluorescent microscope and the latter was first subjected to May-Giemsa staining, and thereafter examined under a light microscope. The results indicated that this cell line does not have pinocytic and phagocytic activities.

As is clear by the above description, the present cell line, although not exhibiting a significant pino- and phagocytosis, may be considered to be a monocytic leukemia cell line in view of the isolation source of the present cell line, the morphological properties and the cytochemical properties. Further, the present cell line is a heretofore unknown novel monocytic leukemia cell line distinctly different from the known monocytic leukemia cell lines, for example, Mono-1 and Mono-1-207 [Virchow Arch. A. Pathol. Anat. Histol., 371, 15 (1976) and 379, 269 (1978)] and THP-1 [Int. J. Cancer 26, 171 (1980)] in the cytochemical properties, the chromosome properties (in particular, the peak of the number of chromosomes), the cell marker (in particular, the cell surface marker and rosette formation) and the cell function (in particular, the presence or absence of phagocytosis).

EP-58 441
0134923

WHAT IS CLAIMED IS;

1. A human endogenous cancer regulatory factor having a molecular weight of 82,000 ± 10,000 and an isoelectric point of pH 6.5 ± 0.5.

2. A process for producing a human endogenous cancer regulatory factor by growing normal human or leukemic cells in a tissue culture medium in the presence of a first stimulant, then adding a second stimulant to the cultured medium to produce the human endogenous cancer regulatory factor, and recovering said regulatory factor from the culture medium.

3. A process according to Claim 2 wherein the leukemic cells are human monocytic leukemia cells.

4. A process according to Claim 3 wherein the human monocytic leukemia cells are human monocyte/macrophage series of leukemia cells.

5. A process according to Claim 4 wherein the human monocyte/macrophage series of leukemia cells are cultured YKBS-7-15 cells (C.N.C.M. I-258).

6. A process according to any of Claims 2 to 5 wherein the tissue culture medium contains a fetal calf serum.

**0134923**

7. A process according to any of Claims 2 to 5 wherein the tissue culture medium is a serum-free synthetic nutrient medium.

8. A process according to any of Claims 2 to 5 wherein the tissue culture medium is the interior of the peritoneal cavity and/or the hypodermic area of a no-human warm-blooded animal.

9. A process according to any of Claims 2 to 8 wherein the first stimulant is a macrophage activating substance.

10. A process according to Claim 9 wherein the macrophage activating substance is 12-0-tetradecanoyl-phorbol-13-acetate.

11. A process according to any of Claims 2 to 8 wherein the first stimulant is a substance capable of activating the reticuloendothelial system.

12. A process according to any of Claims 2 to 11 wherein the second stimulant is an endotoxin produced from Gram-negative or Gram-positive bacteria.

13. A process according to Claim 12 wherein the endotoxin is a lipopolysaccharide produced from Gram-negative bacteria.

0134923

14. A pharmaceutical composition containing the human endogenous cancer regulatory factor according to Claim 1.

15. A pharmaceutical composition according to Claim 14 wherein the composition is a parenterally administered composition.

16. A human monocytic leukemia cell line "YKBS-7-15" and its clones or subclones having cytological Properties of the cell line deposited with the C.N.C.M. as the Deposit Number I-258.

EP-5Ø134923

CLAIMS FOR AUSTRIA:

1. A process for producing a human endogenous cancer regulatory factor having a molecular weight of 82,000 ± 10,000 and an isoelectric point of pH 6.5 ± 0.5 by growing normal human or leukemic cells in a tissue culture medium in the presence of a first stimulant, then adding a second stimulant to the cultured medium to produce the human endogenous cancer regulatory factor, and recovering said regulatory factor from the culture medium.

2. A process according to Claim 1 wherein the leukemic cells are human monocytic leukemia cells.

3. A process according to Claim 2 wherein the human monocytic leukemia cells are human monocyte/macrophage series of leukemia cells.

4. A process according to Claim 3 wherein the human monocyte/macrophage series of leukemia cells are cultured YKBS-7-15 cells (C.N.C.M. I-258).

5. A process according to any of Claims 1 to 4 wherein the tissue culture medium contains a fetal calf serum.

6. A process according to any of Claims 1 to 4 wherein the tissue culture medium is a serum-free synthetic nutrient medium.

7.. A process according to any of Claims 1 to 4 wherein the tissue culture medium is the interior of the peritoneal cavity and/or the hypodermic area of a no-human warm-blooded animal.

8. A process according to any of Claims 1 to 7 wherein the first stimulant is a macrophage activating substance.

9. A process according to Claim 8 wherein the macrophage activating substance is 12-0-tetradecanoyl-phorbol-13-acetate.

10. A process according to any of Claims 1 to 7 wherein the first stimulant is a substance capable of activating the reticuloendothelial system.

11. A process according to any of Claims 1 to 10 wherein the second stimulant is an endotoxin produced from Gram-negative or Gram-positive bacteria.

12. A process according to Claim 11 wherein the endotoxin is a lipopolysaccharide produced from Gram-negative bacteria.

0134923

13. A pharmaceutical composition containing the human endogenous cancer regulatory factor produced according to any of claims 1 to 12.

14. A pharmaceutical composition according to Claim 13 wherein the composition is a parenterally administered composition.

15. A human monocytic leukemia cell line "YKBS-7-15" and its clones or subclones having cytological Properties of the cell line deposited with the C.N.C.M. as the Deposit Number I-258.

Fig. 1

Fig. 2

0134923

Cells

Fig. 3